**Europäisches Patentamt**

(19) **European Patent Office**

**Office européen des brevets**

(11) Publication number : **0 380 175 B1**

(12) ## EUROPEAN PATENT SPECIFICATION

(45) Date of publication of patent specification :
22.09.93 Bulletin 93/38

(51) Int. Cl.⁵ : **C07C 315/02**, C07C 317/22

(21) Application number : **90200152.8**

(22) Date of filing : **22.01.90**

(54) **Process for forming 4,4'-dihydroxydiphenyl sulfone.**

(30) Priority : **27.01.89 US 302514**

(43) Date of publication of application :
**01.08.90 Bulletin 90/31**

(45) Publication of the grant of the patent :
**22.09.93 Bulletin 93/38**

(84) Designated Contracting States :
**DE GB NL**

(56) References cited :
**EP-A- 0 220 004**
**GB-A- 2 030 566**
**GB-A- 2 099 006**
**PATENT ABSTRACTS OF JAPAN, vol. 10, no. 194 (C-358)(2250), 8 July 1986; & JP-A-61 036 253**
**CHEMICAL ABSTRACTS, vol. 85, no. 25, 20 December 1976, page 520, column 2, abstract no. 192367z, Columbus Ohio, USA; & JP-A-76 098 239**

(73) Proprietor : **AKZO N.V.**
**Velperweg 76**
**NL-6824 BM Arnhem (NL)**

(72) Inventor : **Barda, Henry James**
**312 Independence Boulevard**
**No. Brunswick, New Jersey 08902 (US)**
Inventor : **Alwood, David Earl**
**5 Mercer Lane**
**Englishtown, New Jersey 07726 (US)**

(74) Representative : **Schalkwijk, Pieter Cornelis et al**
**AKZO N.V., Patent Department (Dept. CO),**
**P.O. Box 9300**
**NL-6800 SB Arnhem (NL)**

## Description

This invention relates to the preparation of 4,4'-dihydroxydiphenyl sulfone.

Description of the Prior Art

It is known to prepare 4,4'-dihydroxydiphenyl sulfone by reaction between phenol and sulfuric acid (the proportion of phenol being in excess of the stoichiometric proportion of 2 moles of phenol per mole of sulfuric acid) but the desired product is usually accompanied by an unacceptably high proportion of the isomeric 2,4'-dihydroxydiphenyl sulfone as a by-product. The presence of even relatively small amounts of the unwanted 2,4'-isomer in the final product can greatly restrict the field of use of the contaminated 4,4'-dihydroxydiphenyl sulfone product.

In Japanese Patent Publication 51/98239 it has been proposed to carry out the reaction between phenol and sulfuric acid in the presence of a solvent in which 4,4'-dihydroxydiphenyl sulfone is less soluble than 2,4'-dihydroxydiphenyl sulfone, the solvent being gradually distilled from the reaction system. The 4,4'-dihydroxy isomer formed separates and, since the two isomers are in equilibrium, isomerization of the 2,4'-dihydroxy isomer proceeds. This proposed method does, however, require careful control of the reaction conditions to ensure an appropriate balance between the rate of removal of the solvent and the rate of isomerization. Furthermore, the reaction product, after removal of the solvent, may be in the form of a rather intractable solid mass.

British Patent No. 2,030,566 recognizes the problem of undesired 2,4'-isomer in the synthesis procedure alluded to above but directs its efforts at solving the problem after the synthesis reaction has been run by advocating use of heat and a strong acid (e.g., an aqueous mineral acid or a fluoroalkanesulfonic organic acid) to increase the proportion of the desired 4,4'-isomer in a mixture of the 4,4'- and 2,4'- isomers.

While British Patent No. 2,099,006 shows that it is known to use a fluoroalkanesulfonic acid as a reaction solvent or dispersant and as a catalyst, this patent limits its teaching to formation of the polysulfone as contrasted to one of its monomeric sulfone precursors by reaction of phenol and sulfuric acid in an approximate molar ratio of 1:1.

## SUMMARY OF THE PRESENT INVENTION

The present invention is a process for the formation of 4,4'-dihydroxydiphenyl sulfone by reacting phenol and sulfuric acid in the presence of an effective amount of a fluoroalkanesulfonic acid isomerization catalyst for increasing the percentage of 4,4'-isomer in a given unit of reaction time.

## DETAILED DESCRIPTION OF THE PRESENT INVENTION

The procedure for manufacture of 4,4'-dihydroxydiphenyl sulfone in accordance with the instant invention is similar in general outline to the known processes for doing so with the exception of the novel use of the fluoroalkanesulfonic acid isomerization catalyst. Phenol and sulfuric acid are reacted at an approximate molar ratio of 2:1 or more at temperature of from about 150°C to about 200°C in the presence of a suitable hydrocarbon solvent. Water is continuously removed from the system as it is formed.

In accordance with the instant invention, the desired high proportion of 4,4'-isomer in the resulting mixture of 4,4'- and 2,4'- isomers can be achieved in shorter time if a fluoroalkanesulfonic acid isomerization catalyst is included in the reaction medium. Relatively small amounts of this isomerization catalyst can be used (e.g., 0.01 to 1.0%, preferably 0.1% to about 0.2%, by weight of the phenol). The isomerization catalyst has the formula $F_3C(CF_2)_nSO_3H$, where n can be an integer from 0 to 10. A representative acid is trifluoromethanesulfonic acid of the formula $CF_3SO_3H$, where n is 0.

The instant invention is further understood by the Examples which follow.

## EXAMPLES

General Procedure

The apparatus consisted of a 250-ml four neck flask provided with a stirrer containing a vapor tight bearing, a subsurface thermometer, an addition funnel and a fractionating column. The fractionating column had an inside diameter of 2.5 cm and had a silvered vacuum jacket. It was filled to a 28.5 cm height with 0.24 inch (6 mm) stainless steel protruded packing. Above the fractionating column was a vacuum jacketed Barrett moisture trap containing a thermometer immersed in the vapors. Above the Barrett trap was a condenser cooled by a circulating refrigerated bath which was set at 10°C. The stirrer was set at about 190 rpm. The flask was heated in a thermostated silicone oil bath.

The Barrett trap was filled with ISOPAR C solvent. To the flask was added 15.0 grams of ISOPAR C solvent, 120.0 grams of ISOPAR H solvent and 100.0 grams of phenol. When present, phosphoric acid or zinc chloride was also added to the flask. To the addition funnel was added 52.2 grams of 97.06% sulfuric acid. When present, trifluoromethanesulfonic acid was added to the sulfuric acid. The content of the flask was then heated to 100°C, and the sulfuric acid added at 100-110°C followed by a hold period. The addition and hold period together took 20 minutes. The oil bath was then brought to 188-191°C and held at that temperature throughout the reaction. Reflux time, starting when vapors began to condense in the Barrett trap to the removal of the oil bath, was varied. The aqueous phase was periodically drained from the Barrett trap so that it always contained from 1-2 ml. After the first 30-60 minutes of reflux the temperature in the reaction flask was 160-170°C. Typically, 1-3 grams of phenol co-distilled with 19.2-20.2 grams of water. The reaction mixture was then cooled to less than 90°C, most of the ISOPAR solvent replaced by 200 ml of water and stirred at 90°C for 1-2 hours. After cooling to 30°C, the slurry was filtered, washed three times with 120 ml of water, and dried to constant weight.

The product was analyzed by high pressure liquid chromatography. The 4,4'-DHS is reported as a percent of the total DHS content (isomer distribution), while the trimer, an impurity, is reported as a percent of the DHS and trimer content (assay).

### EXAMPLES 1-4

#### Containing 0.0 gram of trifluoromethanesulfonic acid

| Example | 1 | 2 | 3 | 4 |
|---|---|---|---|---|
| Reflux time, hours | 4.25 | 8.0 | 12.0 | 16.0 |
| 4,4'-DHS,% (isomer distribution) | 92.49 | 96.50 | 97.65 | 98.12 |
| Trimer,% (assay) | 1.37 | 1.48 | 1.35 | 1.24 |
| Yield,% | 89.30 | 91.70 | 93.50 | 94.04 |

### EXAMPLES 5-8

#### Containing 0.1 gram trifluoromethanesulfonic acid

| Example | 5 | 6 | 7 | 8 |
|---|---|---|---|---|
| Reflux time, hours | 4.25 | 6.0 | 8.0 | 10.0 |
| 4,4'-DHS,% (isomer distribution) | 95.59 | 97.25 | 98.04 | 98.13 |
| Trimer,% (assay) | 1.22 | 1.44 | 1.47 | 1.09 |
| Yield,% | 91.20 | 92.70 | 92.20 | 93.70 |

### EXAMPLES 9-12

#### Containing 0.2 gram trifluoromethanesulfonic acid

| Example | 9 | 10 | 11 | 12 |
|---|---|---|---|---|
| Reflux time, hours | 4.25 | 5.0 | 6.0 | 8.0 |
| 4,4'-DHS,% (isomer distribution) | 96.30 | 97.06 | 98.08 | 98.06 |
| Trimer,% (assay) | 1.38 | 1.38 | 1.12 | 1.19 |
| Yield,% | 92.50 | 91.80 | 93.35 | 93.89 |

## EXAMPLE 13

### Containing 0.3 gram trifluoromethanesulfonic acid

| Example | 13 |
|---|---|
| Reflux time, hours | 4.25 |
| 4,4'-DHS,% (isomer distribution) | 96.70 |
| Trimer,% (assay) | 1.56 |
| Yield,% | 92.89 |

## EXAMPLES 14-15

### Containing phosphoric acid or zinc chloride

| Example | 14 | 15 |
|---|---|---|
| 85% phosphoric acid, grams | 5.0 | - |
| Zinc chloride, grams | - | 5.0 |
| Reflux time, hours | 4.25 | 4.25 |
| 4,4'-DHS,% (isomer distribution) | 85.25 | 89.85 |
| Trimer,% (assay) | 2.41 | 0.69 |
| Yield,% | 81.00 | 74.90 |

The results of Examples 1 to 12 were plotted on a graph. It showed three curves which plateaued at or about a 98% 4,4'-DHS isomer distribution. It also showed that as the trifluoromethanesulfonic acid content was increased it took fewer hours of reflux to obtain a 98% 4,4'-DHS isomer distribution. Table I below emphasizes this point.

## TABLE I

### Reflux time to Reach 98% 4,4'-DHS Isomer Distribution

| Example | Trifluoromethanesulfonic Acid, grams | Hours |
|---|---|---|
| 4 | 0.0 | 16 |
| 7 | 0.1 | 8 |
| 11 | 0.2 | 6 |

Table II below illustrates the fact that for a given reflux time the 4,4'-DHS isomer distribution increased with trifluoromethanesulfonic acid content.

## TABLE II

### Isomer Distribution as a Function of Trifluoromethanesulfonic Acid Content After 4.25 hours of Reflux

| Example | Trifluoromethanesulfonic Acid, grams | 4,4'-DHS,% |
|---|---|---|
| 1 | 0.0 | 92.49 |
| 2 | 0.1 | 95.59 |
| 9 | 0.2 | 96.30 |
| 13 | 0.3 | 96.70 |

The foregoing Examples are presented for illustrative purposes only and should not therefore be construed in a limiting sense. The scope of protection which is sought is set forth in the claims which follow.

## Claims

1. A process for the formation of 4,4'-dihydroxydiphenyl sulfone by reacting phenol and sulfuric acid in the presence of a catalytically effective amount of a fluoroalkanesulfonic acid isomerization catalyst to increase the rate of formation of 4,4'-dihydroxydiphenyl sulfone from the reaction.

2. A process as claimed in Claim 1 wherein the fluoroalkanesulfonic acid catalyst is of the formula $F_3C(CF_2)_nSO_3H$ where n is an integer from 0 to 10.

3. A process as claimed in Claim 1 wherein the fluoroalkanesulfonic acid catalyst is fluoromethanesulfonic acid.

4. A process as claimed in Claim 1 wherein the amount of catalyst ranges from about 0.01% to about 1%, by weight of the phenol.

5. A process as claimed in Claim 3 wherein the amount of catalyst ranges from about 0.01% to about 1%, by weight of the phenol.

6. A process as claimed in Claim 3 wherein the amount of catalyst ranges from about 0.01% to about 0.2%, by weight of the phenol.

## Patentansprüche

1. Verfahren zur Herstellung von 4,4'-Dihydroxydiphenylsulfon durch Umsetzung von Phenol und Schwefelsäure in Gegenwart einer katalytisch wirksamen Menge eines Fluoralkansulfonsäure-Isomerisierungskatalysators zur Erhöhung der Bildungsrate des 4,4'-Dihydroxydiphenylsulfons bei der Umsetzung.

2. Verfahren nach Anspruch 1, bei welchem der Fluoralkansulfonsäurekatalysator der Formel $F_3C(CF_2)nSO_3H$ entspricht, worin n eine ganze Zahl von 0 bis 10 ist.

3. Verfahren nach Anspruch 1, bei welchem der Fluralkansulfonsäurekatalysator Fluormethansulfonsäure ist.

4. Verfahren nach Anspruch 1, bei welchem der Anteil des Katalysators im Bereich von etwa 0,01 bis etwa 1 % des Phenolgewichts liegt.

5. Verfahren nach Anspruch 3, bei welchem der Katalysatoranteil im Bereich von etwa 0,01 bis etwa 1 % des Phenolgewichts liegt.

6. Verfahren nach Anspruch 3, bei welchem der Katalysatoranteil im Bereich von etwa 0,01 bis etwa 0,2 % des Phenolgewichts liegt.

## Revendications

1. Procédé de formation de 4,4'-dihydroxydiphénylsulfone par réaction de phénol et d'acide sulfurique en présence d'une quantité catalytiquement efficace d'un catalyseur d'isomérisation de type acide fluoroalcanesulfonique destiné à augmenter le taux de formation de 4,4'-dihydroxydiphénylsulfone de la réaction.

2. Procédé selon la revendication 1, dans lequel la formule du catalyseur de type acide fluoroalcanesulfonique est $F_3C(CF_2)_nSO_3H$ dans laquelle n est un nombre entier compris entre 0 et 10.

3. Procédé selon la revendication 1, dans lequel le catalyseur de type acide fluoroalranesulfonique est l'acide fluorométhanesulfonique.

4. Procédé selon la revendication 1, dans lequel la quantité de catalyseur se situe dans une gamme d'environ 0,01 % à environ 1 % par rapport au poids du phénol.

5. Procédé selon la revendication 3, dans lequel la quantité de catalyseur se situe dans une gamme d'environ 0,01 % à environ 1 % par rapport au poids du phénol.

6. Procédé selon la revendication 3, dans lequel la quantité de catalyseur se situe dans une gamme d'environ 0,01 % à environ 0,2 % par rapport au poids du phénol.